(19) Europäisches Patentamt
European Patent Office
Office européen des brevets

(11) EP 2 285 322 B1

(12) EUROPÄISCHE PATENTSCHRIFT

(45) Veröffentlichungstag und Bekanntmachung des Hinweises auf die Patenterteilung:
**25.02.2015 Patentblatt 2015/09**

(21) Anmeldenummer: **09730588.2**

(22) Anmeldetag: **28.03.2009**

(51) Int Cl.:
**A61F 9/009** *(2006.01)*    **A61F 9/01** *(2006.01)*

(86) Internationale Anmeldenummer:
**PCT/EP2009/002289**

(87) Internationale Veröffentlichungsnummer:
**WO 2009/124668 (15.10.2009 Gazette 2009/42)**

(54) **VERFAHREN ZUM ERZEUGEN VON STEUERDATEN FÜR DIE AUGENCHIRURGIE SOWIE AUGENCHIRURGISCHE BEHANDLUNGSVORRICHTUNG**

METHOD FOR GENERATING CONTROL DATA FOR EYE SURGERY AND EYE-SURGICAL TREATMENT DEVICE

PROCÉDÉ DE PRODUCTION DE DONNÉES DE COMMANDE POUR LA CHIRURGIE DE L'OEIL ET DISPOSITIF DE TRAITEMENT POUR LA CHIRUGIE DE L'OEIL

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **04.04.2008 DE 102008017293**

(43) Veröffentlichungstag der Anmeldung:
**23.02.2011 Patentblatt 2011/08**

(73) Patentinhaber: **Carl Zeiss Meditec AG**
**07745 Jena (DE)**

(72) Erfinder:
• **BISCHOFF, Mark**
**07749 Jena (DE)**
• **STOBRAWA, Gregor**
**07743 Jena (DE)**

(74) Vertreter: **Geyer, Fehners & Partner**
**Patentanwälte**
**Perhamerstrasse 31**
**80687 München (DE)**

(56) Entgegenhaltungen:
EP-A- 1 364 632         WO-A-2005/011547
DE-A1-102006 046 370

## Beschreibung

Die Erfindung bezieht sich auf ein Verfahren zum Erzeugen von Steuerdaten für eine augenchirurgische Behandlungsvorrichtung, welche mittels einer Lasereinrichtung in der Augen-Hornhaut Gewebeschichten trennt, wobei im Betrieb der Lasereinrichtung ein eine Kontaktfläche aufweisendes Kontaktglas die Hornhaut in die Form der Kontaktfläche verformt, wozu die Kontaktfläche zuerst mit einem Kontaktflächenscheitel auf einen Hornhautscheitel gesetzt und dann zur Verformung der Hornhaut gegen diese gedrückt wird, wobei das Verfahren folgende Schritte umfaßt: die Steuerdaten für die Lasereinrichtung werden so erzeugt, daß sie Koordinaten von in der Hornhaut liegenden Zielpunkten für die Lasereinrichtung vorgeben, und bei der Erzeugung der Zielpunkt-Koordinaten wird die Verformung der Hornhaut berücksichtigt, welche während des Betriebs der Lasereinrichtung bedingt durch das Kontaktglas vorliegt.

Die Erfindung bezieht sich weiter auf eine augenchirurgische Behandlungsvorrichtung, welche aufweist: eine Lasereinrichtung zur Trennung von Gewebeschichten der Augen-Hornhaut, ein eine Kontaktfläche aufweisendes Kontaktglas, das im Betrieb der Lasereinrichtung die Hornhaut in die Form der Kontaktfläche verformt, wozu die Kontaktfläche zuerst mit einem Kontaktflächenscheitel auf einen Hornhautscheitel gesetzt und dann zur Verformung der Hornhaut gegen diese gedrückt wird, ein Steuergerät, das Steuerdaten für die Lasereinrichtung so erzeugt, daß sie Koordinaten von in der Hornhaut liegenden Zielpunkt für die Lasereinrichtung vorgeben, und bei der Erzeugung der Zielpunkt-Koordinaten die Verformung der Hornhaut berücksichtigt, welche während des Betriebs der Lasereinrichtung bedingt durch das Kontaktglas vorliegt.

Der klassische Weg um Fehlsichtigkeit des menschlichen Auges zu korrigieren, ist seit langer Zeit die Brille. Mittlerweile wird jedoch zunehmend auf refraktive Chirurgie zurückgegriffen, die durch Veränderungen der Augenhornhaut eine Fehlsichtigkeitskorrektur bewirkt. Ziel aller Operationsmethoden ist es dabei, die Hornhaut gezielt zu verändern, um so die Lichtbrechung zu beeinflussen. Hierfür sind unterschiedliche Operationsmethoden bekannt. Am weitverbreitesten ist die sogenannte Laser-Insitu-Keratomileusis, die auch LASIK abgekürzt wird. Dabei wird zuerst eine Hornhautlamelle von der Hornhautoberfläche einseitig gelöst und zur Seite geklappt. Das Lösen dieser Lamelle kann mittels eines mechanischen Mikrokeratoms erfolgen, oder auch mittels eines sogenannten Laserkeratoms, wie es z.B. Intralase Corp., Irvine, USA vertreibt.

Letzteres erzeugt in der Hornhaut durch Laserstrahlung eine Schnittfläche. Dabei laufen im Gewebe zeitlich hintereinander mehrere Prozesse ab, die durch die Laserstrahlung initiiert werden. Liegt die Leistungsdichte der Strahlung über einen Schwellwert, kommt es zu einem optischen Durchbruch, der in der Hornhaut eine Plasmablase erzeugt. Die Plasmablase wächst nach Entstehen des optischen Durchbruches durch sich ausdehnende Gase. Wird der optische Durchbruch nicht aufrecht erhalten, so wird das in der Plasmablase erzeugte Gas vom umliegenden Material aufgenommen und die Blase verschwindet wieder. Es sind auch gewebetrennende Effekte möglich, die ohne Plasmablase wirken. Der Einfachheit halber werden all solche Prozesse hier unter dem Begriff "optischer Durchbruch" zusammengefaßt, d.h. dieser Begriff soll nicht nur den optischen Durchbruch sondern auch die daraus resultierenden Wirkungen in der Hornhaut einschließen.

Zur Erzeugung des optischen Durchbruches wird die Laserstrahlung gepulst angewendet, wobei die Pulslänge unter 1 ps liegt. Dadurch wird die zur Auslösung eines optischen Durchbruches nötige Leistungsdichte für den jeweiligen Puls nur in einem engen räumlichen Gebiet erreicht. Die US 5984916 zeigt diesbezüglich deutlich, daß der räumliche Bereich des optischen Durchbruches (in diesem Fall der erzeugten Wechselwirkung) stark von der Pulsdauer abhängt. Eine hohe Fokussierung des Laserstrahls in Kombination mit den erwähnten kurzen Pulsen erlaubt es damit, den optischen Durchbruch punktgenau in der Hornhaut einzusetzen.

Zur Erzeugung der dünnen Lamelle wird nun mit dem Laserkeratom eine Serie optischer Durchbrüche an vorbestimmten Stellen so erzeugt, daß eine Schnittfläche ausgebildet wird, welche die Lamelle von der darunterliegenden Hornhaut löst.

Nachdem die Lamelle gelöst und zur Seite geklappt wurde, ist bei der LASIK-Operation die Anwendung eines Excimer-Lasers vorgesehen, der das nun freiliegende Hornhautgewebe durch Ablation abträgt. Nachdem auf diese Art und Weise in der Hornhaut liegendes Volumen verdampft wurde, wird die Hornhautlamelle wieder auf den ursprünglichen Platz zurückgeklappt. Die bereits in der Anwendung befindliche LASIK-Methode, die, soweit ein Laserkeratom zum Einsatz kommt, als auch als fs-LASIK bezeichnet wird, legt somit eine deckelförmige Hornhautlamelle frei, klappt diese ab und ablatiert das freigelegte Gewebe mit einem Ablationslaser.

Es ist im Stand der Technik auch erwähnt, daß die Fehlsichtigkeitskorrektur dadurch erzeugt wird, daß mittels der gepulsten Laserstrahlung ein linsenförmiges Teilvolumen im Hornhautgewebe isoliert wird. Eine entsprechende Schilderung findet sich beispielsweise in der WO 2005/011545 A1. Entsprechend sind Geräte jedoch am Markt noch nicht verfügbar.

Die Präzision, mit der die Schnittfläche erzeugt wird, ist natürlich für die optische Korrektur letztlich entscheidend. Dies gilt ganz besonders für weitergebildete laserchirurgische Fehlsichtigkeitskorrekturverfahren, bei denen ein in der Hornhaut liegendes Volumen durch eine dreidimensionale Schnittfläche isoliert und so entfernbar gemacht wird. Anders als beim Laserkeratom ist dann die Lage der Schnittfläche direkt relevant für optische Korrektur. Bei der herkömmlichen LASIK-Methode ist dagegen ausschließlich die Präzision, mit der die Laserablation betrieben wird, für die Güte der optischen Korrektur wichtig, was man schon daran erkennt, daß die Erzeugung der Hornhautlamelle in einer großen Vielzahl von Operationen mit einem vergleichsweise grob arbeitenden mechanischen Messer praktiziert wird bzw. wurde.

Weiter ist aus der WO 2005/011547 A1 bekannt, daß bei laserchirurgischen Vorrichtungen ein Kontaktglas verwendet werden kann, an das die Augenhornhaut angepreßt wird. Dieses Kontaktglas dient dazu, der Hornhaut eine feste definierte Form zu geben und zugleich das Auge zu fixieren. Beim Anpressen an das Kontaktglas kommt es somit zu einer Deformation der Hornhaut, die bei der Bestimmung der Koordinaten der Zielpunkte dadurch berücksichtigt wird, daß Anpressen und Deformation in einer Koordinatentransformation berücksichtigt wird, die in der WO-Veröffentlichung als "Anpreßtransformation" bezeichnet ist und einer Verschiebung der Zielpunkte Rechnung trägt. In der Druckschrift werden Transformationsgleichungen für den Fall einer Kombination aus sphärischem Kontaktglas und sphärischer Hornhautvorderfläche gegeben. Die Veröffentlichung erwähnt zwar, daß weitere Ergänzungen mittels Korrekturtermen möglich sind, schildert solche jedoch nicht. Somit gelten die dort genannten Transformationsgleichungen ausschließlich für sphärische Kontaktgläser und sphärische Hornhautvorderflächen, die jedoch nicht immer gegeben sind.

Der Erfindung liegt deshalb die Aufgabe zugrunde, ein verbessertes Verfahren zum Erzeugen von Steuerdaten, eine verbesserte Behandlungseinrichtung sowie ein verbessertes Behandlungsverfahren anzugeben, die auch jenseits der von der WO 2005/01157 A1 gegebenen Rahmenbedingungen zuverlässig die kontaktglasbedingte Verformung der Hornhaut berücksichtigen können, somit zu einem präziseren laserchirurgischen Operationsergebnis führen.

Diese Aufgabe wird erfindungsgemäß gelöst mit einem Verfahren zum Erzeugen von Steuerdaten für eine augenchirurgische Behandlungsvorrichtung, welche mittels einer Lasereinrichtung in der Augen-Hornhaut Gewebeschichten trennt, wobei im Betrieb der Lasereinrichtung ein eine Kontaktfläche aufweisendes Kontaktglas die Hornhaut in die Form der Kontaktfläche verformt, wozu die Kontaktfläche gegen die Hornhaut gedrückt wird, wobei das Verfahren folgende Schritte umfaßt: die Steuerdaten für die Lasereinrichtung werden so erzeugt, daß sie Koordinaten von in der Hornhaut liegenden Zielpunkten für die Lasereinrichtung vorgeben, und bei der Erzeugung der Zielpunkt-Koordinaten wird die Verformung der Hornhaut berücksichtigt, welche während des Betriebs der Lasereinrichtung bedingt durch das Kontaktglas vorliegt, wobei bei der Berücksichtigung der Verformung folgende Schritte ausgeführt werden, um eine verformungsbedingte Verschiebung eines Punktes P in der unverformten Hornhaut zu ermitteln:

1a) zum Punkt P wird ein Punkt O auf einer Bezugsfläche V der unverformten Hornhaut ermittelt, der so liegt, daß die durch den Punkt O laufende Flächennormale durch den Punkt P geht, wobei die Bezugsfläche entweder die Vorderfläche selbst oder eine durch radiale Kontraktion der Vorderfläche um eine Biegelinienverschiebung L erhaltene Fläche ist,

1b) ein Abstand $\overline{SO}$ zwischen einem Scheitelpunkt S der Bezugsfläche und dem Punkt O wird ermittelt, wobei der Abstand $\overline{SO}$ die Bogenlänge auf der Bezugsfläche ist,

1c) es wird ein Punkt O' auf einer Kontaktglas-Bezugsfläche ermittelt, der im Abstand $\overline{SO}$ vom Kontaktflächenscheitel liegt, wobei die Kontaktglas-Bezugsfläche entweder die Kontaktfläche selbst oder eine durch radiale Kontraktion der Kontaktfläche um die Biegelinienverschiebung und/oder eine Dicke F' eines Flüssigkeitsfilms auf der Hornhaut erhaltene Fläche ist,

1d) auf einer Flächennormalen im Punkt O' wird ein Punkt P' ermittelt, der denselben Abstand von O' hat, wie der Punkt P vom Punkt O, und

1e) der Punkt P' wird als durch die Verformung verschobener Punkt P verwendet; und/oder bei der Berücksichtigung der Verformung folgende Schritte ausgeführt werden, um eine relaxationsbedingte Verschiebung eines Punktes Q' in der verformten Hornhaut zu ermitteln:

2a) zum Punkt Q' wird ein Punkt O' auf der Kontaktglas-Bezugsfläche ermittelt, der so liegt, daß die durch den Punkt O' laufende Flächennormale durch den Punkt Q' geht,

2b) ein Abstand $\overline{SO'}$ zwischen dem Scheitelpunkt S und dem Punkt O' wird ermittelt, wobei der Abstand $\overline{SO'}$ die Bogenlänge auf der Kontaktglas-Bezugsfläche ist,

2c) es wird ein Punkt O auf der Bezugsfläche ermittelt, der im Abstand $\overline{SO'}$ vom Scheitelpunkt S liegt,

2d) auf einer Flächennormalen im Punkt O wird ein Punkt Q ermittelt, der denselben Abstand von O hat, wie der Punkt Q' vom Punkt O', und

2e) der Punkt Q wird als durch die Relaxation verschobener Punkt Q' verwendet.

Die Aufgabe wird auch gelöst mit einer augenchirurgischen Behandlungsvorrichtung, welche aufweist: eine Lasereinrichtung zur Trennung von Gewebeschichten der Augen-Hornhaut, ein eine Kontaktfläche aufweisendes Kontaktglas, das im Betrieb der Lasereinrichtung die Hornhaut in die Form der Kontaktfläche verformt, wozu die Kontaktfläche zuerst mit einem Kontaktflächenscheitel auf einen Hornhautscheitel gesetzt und dann zur Verformung der Hornhaut gegen diese gedrückt wird, ein Steuergerät, das Steuerdaten für die Lasereinrichtung so erzeugt, daß sie Koordinaten von in der Hornhaut liegenden Zielpunkt für die Lasereinrichtung vorgeben, und bei der Erzeugung der Zielpunkt-Koordinaten die Verformung der Hornhaut berücksichtigt, welche während des Betriebs der Lasereinrichtung bedingt durch das Kontaktglas vorliegt, wobei das Steuergerät das genannte Verfahren ausführt.

Die Erfindung geht also nicht mehr davon aus, daß die Augenhornhaut sphärisch ist, bzw. daß die Biegelinie strikt auf der Oberfläche der Augenhornhaut liegt und die Dicke des Tränenfilms vernachlässigbar ist. Durch den erfindungsgemäßen Ansatz werden Unterschiede zwischen der beabsichtigten und der erzielten Schnittführung vermieden, insbesondere an Randzonen des Bearbeitungsgebietes. Die gesteigerte Präzision hat dabei die Konsequenz, daß es möglich ist, auch mit vergleichsweise stärker gekrümmten Kontaktgläsern bzw. Kontaktflächen zu arbeiten, wohingegen der Stand der Technik bislang dahin tendierte, die Kornea möglichst zu applanieren, obwohl dies aus verschiedenen Gründen, insbesondere wegen einer Erhöhung des Augeninnendrucks, nachteilig ist.

Die Erfindung hat dabei den Vorteil, daß sie auch bei beliebiger, insbesondere nicht-sphärischer oder nicht-rotationssymmetrischer Kontaktglasform angewendet werden kann, da jeder Punkt P in der freien Hornhaut hinsichtlich seiner Koordinaten zu einem Punkt P' transformiert wird, der bei verformtem, d.h. an die Kontaktfläche angelegtem Auge dem Punkt P entspricht. Die Verfahrensschritte der ersten Variante beschreiben dabei, wie für jeden Punkt, der in der unverformten Hornhaut gegeben ist, die entsprechenden Koordinaten nach Verformung der Hornhaut ermittelt werden. Dies stellt die sogenannte Hin-Transformation dar. Ein analoger Ablauf führt aber auch zur Rück-Transformation wird für einen Punkt, dessen Koordinaten in der verformten Hornhaut bekannt sind, die entsprechenden Koordinaten in der unverformten Hornhaut, also nach Relaxation durch Abnahme des Kontaktglases bestimmt werden sollen. Anspruch 1 sowie die entsprechenden einbezogenen oder parallelen Ansprüche definiert in den Schritten 1a) - 1e) die Hin-Transformation, in den Schritten 2a) - 2e) die Rück-Transformation. Eine von beiden wird erfindungsgemäß ausgeführt.

Das Verfahren der ersten Variante hat den Vorteil, daß es allgemein für beliebige Oberflächen anwendbar ist.

Ein weiterer Einfluß, der zu einer Abweichung der tatsächlichen Lage der Schnittfläche von der gemäß den Steuerdaten zur erwartenden Lage führen kann, ist die Dicke eines Flüssigkeitsfilms, welcher auch im verformten Zustand der Hornhaut einen von Flüssigkeit gefüllten Spalt zwischen Hornhautvorderfläche und Kontaktfläche des Kontaktglases zur Folge hat. Diese Flüssigkeit ist nicht zwingend gleichzusetzen mit einem natürlichen Tränenfilm, da üblicherweise bei einem Eingriff das Auge durch ein lokales Anästhetikum (in Tropfenform) betäubt wird und künstliche Tränenflüssigkeit aufgebracht wird. Es liegt deshalb nicht automatisch die natürliche Tränenfilmdicke vor. Zudem ändert sich durch das Anpressen und die Verformung die Flüssigkeitsdicke zusätzlich.

Weiter kann auch optional der Tatsache Rechnung getragen werden, daß die Biegelinie, also die neutrale Phase der Verformung, bei der Verformung der Hornhaut nicht exakt auf der Hornhautvorderfläche verläuft, sondern in die Hornhaut hinein, also nach innen, verschoben sein kann. Diese Verschiebung kann aus experimentellen Daten ermittelt werden. In einer vereinfachten Variante wird davon ausgegangen, daß die Biegelinie auf der Mittendicke der Hornhaut liegt, die Verschiebung also der halben Hornhautdicke entspricht.

Die Erfindung wird nachfolgend unter Bezugnahme auf die Zeichnung beispielhalber noch näher erläutert. In der Zeichnung zeigt:

Fig.1 eine Schemadarstellung einer Behandlungsvorrichtung bzw. eines Behandlungsgerätes zur Fehlsichtigkeitskorrektur,

Fig. 1a eine Schemadarstellung hinsichtlich des Aufbaus des Behandlungsgerätes der Fig. 1,

Fig. 2 eine Prinzipdarstellung zur Einbringung gepulster Laserstrahlung in das Auge bei der Fehlsichtigkeitskorrektur mit dem Behandlungsgerät der Fig. 1,

Fig. 3 eine weitere Schemadarstellung des Behandlungsgerätes der Fig. 1,

Fig. 4 eine schematische Darstellung zur Erläuterung der Funktion eines Kontaktglases im Behandlungsgerät der Fig. 1,

Fig. 5 bis 7 schematische Darstellungen hinsichtlich der Wirkungen des Kontaktglases durch Verformung der Augenhornhaut und

Fig. 8 eine schematische Darstellung des Ablaufes bei der Vorbereitung und Durchführung einer Fehlsichtigkeitskorrektur.

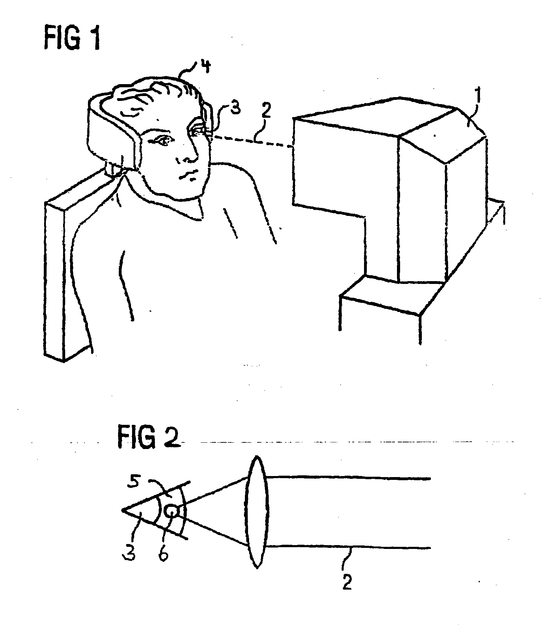

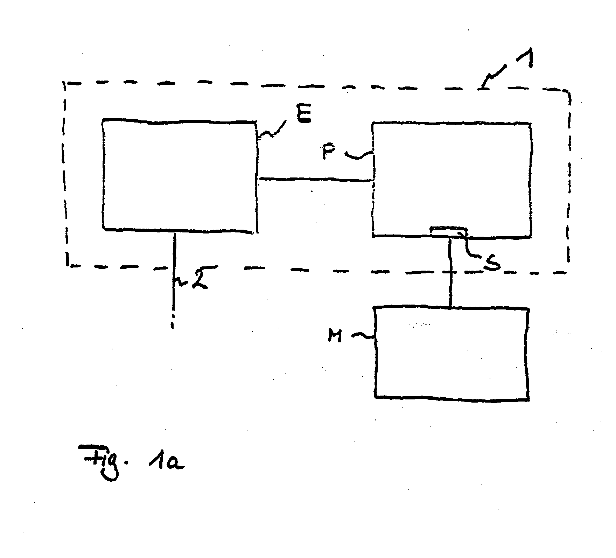

Figur 1 zeigt ein Behandlungsgerät 1 für ein augenchirurgisches Verfahren, das dem in der EP 1159986 A1 bzw. der US 5549632 beschriebenen ähnelt. Das Behandlungsgerät 1 bewirkt mittels ein Behandlungs-Laserstrahlung 2 eine Fehlsichtigkeitskorrektur an einem Auge 3 eines Patienten 4. Die Fehlsichtigkeit kann Hyperopie, Myopie, Presbyopie, Astigmatismus, gemischten Astigmatismus (Astigmatismus, bei dem in einer Richtung Hyperopie und in einer rechtwinklig dazu liegenden Richtung Myopie vorliegt), asphärische Fehler und Abberationen höherer Ordnung umfassen. Die Behandlungs-Laserstrahlung 2 wird in der beschriebenen Ausführungsform als gepulster in das Auge 3 fokussierter Laserstrahl aufgebracht. Die Pulsdauer liegt dabei z.B. im Femtosekundenbereich, und die Laserstrahlung 2 wirkt mittels nicht-linearer optischer Effekte in der Hornhaut. Der Laserstrahl weist z.B. 50 bis 800 fs kurze Laserpulse (bevorzugt 100 - 400 fs) mit einer Pulswiederholfrequenz zwischen 10 und 500 kHz auf. Die Baugruppen des Gerätes 1 werden im beschriebenen Ausführungsbeispiel von einer integrierten Steuereinheit gesteuert, die aber natürlich auch eigenständig ausgebildet sein kann.

Vor dem Einsatz des Behandlungsgerätes wird die Fehlsichtigkeit des Auges 3 mit einer oder mehreren Meßeinrichtungen vermessen.

Figur 1a zeigt schematisch das Behandlungsgerät 1. Es weist in dieser Variante mindestens zwei Einrichtungen oder Module auf. Eine Lasereinrichtung E gibt den Laserstrahl 2 auf das Auge 3 ab. Der Betrieb der Lasereinrichtung E erfolgt dabei vollautomatisch, d.h. die Lasereinrichtung E startet auf ein entsprechendes Startsignal hin die Ablenkung des Laserstrahls 2 und erzeugt dabei Schnittflächen, die auf noch zu beschreibende Art und Weise aufgebaut sind und ein Volumen in der Augenhornhaut isolieren. Die den Betrieb erforderlichen Steuerdaten empfängt die Lasereinrichtung E zuvor von einer Planungseinrichtung P als Steuerdatensatz über nicht näher bezeichnete Steuerleitungen. Die Übertragung findet vor dem Betrieb der Lasereinrichtung E statt. Natürlich kann Kommunikation auch drahtlos erfolgen. Alternativ zu einer direkten Kommunikation ist es auch möglich, die Planungseinheit P räumlich getrennt von der Lasereinrichtung E anzuordnen und einen entsprechenden Datenübertragungskanal vorzusehen.

Vorzugsweise wird der Steuerdatensatz zum Behandlungsgerät 1 übertragen und weiter vorzugsweise ist ein Betrieb der Lasereinrichtung E gesperrt, bis an der Lasereinrichtung E ein gültiger Steuerdatensatz vorliegt. Ein gültiger Steuerdatensatz kann ein Steuerdatensatz sein, der prinzipiell zur Verwendung mit der Lasereinrichtung E der Behandlungsvorrichtung 1 geeignet ist. Zusätzlich kann die Gültigkeit aber auch daran geknüpft werden, daß weitere Prüfungen bestanden werden, beispielsweise ob im Steuerdatensatz zusätzlich niedergelegte Angaben über das Behandlungsgerät 1, z. B. eine Geräteseriennummer, oder den Patienten, z. B. eine Patientenidentifikationsnummer, mit anderen Angaben übereinstimmen, die beispielsweise an der Behandlungsvorrichtung ausgelesen oder separat eingegeben wurden, sobald der Patient in der korrekten Stellung für den Betrieb der Lasereinrichtung E ist.

Die Planungseinheit P erzeugt den Steuerdatensatz, der der Lasereinrichtung E zur Ausführung der Operation zur Verfügung gestellt wird, aus Meßdaten und Fehlsichtigkeitsdaten, die für das zu behandelnde Auge ermittelt wurden. Sie werden der Planungseinheit P über eine Schnittstelle S zugeführt und stammen im dargestellten Ausführungsbeispiel aus einer Meßeinrichtung M, die das Auge des Patienten 4 zuvor vermessen hat. Natürlich kann die Meßeinrichtung M auf beliebige Art und Weise die entsprechenden Meß- und Fehlsichtigkeitsdaten an die Planungseinrichtung P übermitteln.

Die Wirkungsweise des Laserstrahls 2 ist in Figur 2 schematisch angedeutet. Der Behandlungs-Laserstrahl 2 wird mittels einer nicht näher bezeichneten Optik in die Hornhaut 5 des Auges 6 fokussiert. Dadurch entsteht in der Hornhaut 5 ein Fokus, der einen Spot 6 überdeckt und in dem die Laserstrahlungsenergiedichte so hoch ist, daß in Kombination mit der Pulslänge ein nicht-linearer Effekt im Auge auftritt. Beispielsweise kann jeder Puls der gepulsten Laserstrahlung 2 am jeweiligen Spot 6 einen optischen Durchbruch in der Augenhornhaut 5 erzeugen, welcher wiederum eine in Figur 2 schematisch angedeutete Plasmablase initiiert. Dadurch wird mittels dieses Laserpulses in der Hornhaut 5 Gewebe getrennt. Bei Entstehung einer Plasmablase umfaßt die Gewebeschichttrennung ein größeres Gebiet, als den Spot 6, welchen der Fokus der Laserstrahlung 2 überdeckt, obwohl die Bedingungen zur Erzeugung des Durchbruches nur im Fokus erreicht werden. Damit von jedem Laserpuls ein optischer Durchbruch von jedem Laserpuls erzeugt wird, muß die Energiedichte, d.h. die Fluence der Laserstrahlung oberhalb eines gewissen, pulslängenabhängigen Schwellwertes liegen. Dieser Zusammenhang ist dem Fachmann beispielsweise aus der DE 69500997 T2 bekannt.

Alternativ kann ein gewebetrennender Effekt durch die gepulste Laserstrahlung auch dadurch erzeugt werden, daß mehrere Laserstrahlungspulse im einen Bereich abgegeben werden, wobei sich für mehrere Laserstrahlungspulse die Spots 6 überlappen. Es wirken dann mehrere Laserstrahlungspulse zusammen, um einen gewebetrennenden Effekt zu erreichen.

Die Art der Gewebetrennung, die das Behandlungsgerät 1 einsetzt, ist jedoch für diese Beschreibung nicht weiter relevant. Wesentlich ist lediglich, daß das Behandlungsgerät 1 im Gewebe eine Schnittfläche ausbildet, deren Form durch Punkte im Gewebe charakterisiert ist. Diese Punkte können z. B. Zielpunkte für eine Fokuslage vorgeben, an denen ein oder mehrere Laserpuls(e) abgegeben wird (werden). Für die nachfolgend erläuterten Verfahren und Vorrichtungen ist die Definition von Punkten im Gewebe/Material von Bedeutung und wird noch näher beschrieben werden. Diese Beschreibung stützt sich lediglich exemplarisch darauf, daß die Punkte Zielpunkte für gepulste Laserstrahlung sind.

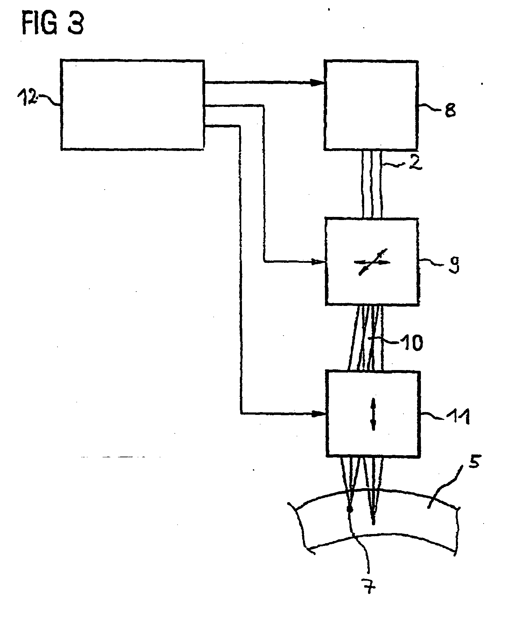

Um eine Fehlsichtigkeitskorrektur auszuführen, wird mittels der gepulsten Laserstrahlung aus einem Gebiet innerhalb der Hornhaut 5 Material entfernt, indem dort Gewebeschichten getrennt werden, die das Material isolieren und dann eine Materialentnahme ermöglichen. Die Materialentfernung bewirkt eine Volumenänderung in der Hornhaut, welche eine Änderung der optischen Abbildungswirkung der Hornhaut 5 zur Folge hat, die genau so bemessen ist, daß damit die zuvor ermittelte Fehlsichtigkeit möglichst korrigiert ist/wird. Zur Isolierung des zu entfernenden Volumens wird der Fokus der Laserstrahlung 2 auf Zielpunkte in der Hornhaut 5 gerichtet, in der Regel in einem Bereich, der unterhalb des Epithels und der Bowman'schen Membran sowie oberhalb der Decemetschen Membran und des Endothels liegt. Das Behandlungsgerät 1 weist dazu einen Mechanismus zum Verstellen der Lage des Fokus der Laserstrahlung 2 in der Hornhaut 5 auf. Dies ist schematisch in Figur 3 gezeigt.

In Figur 3 sind Elemente des Behandlungsgeräts 1 nur insoweit eingetragen, als sie zum Verständnis der Fokusverstellung erforderlich sind. Die Laserstrahlung 2 wird, wie bereits erwähnt, in einem Fokus 7 in der Hornhaut 5 gebündelt, und die Lage des Fokus 7 in der Hornhaut wird verstellt, so daß zur Schnittflächenerzeugung an verschiedenen Stellen fokussiert Energie aus Laserstrahlungspulsen in das Gewebe der Hornhaut 5 eintragen wird. Die Laserstrahlung 2 wird von einem Laser 8 als gepulste Strahlung bereitgestellt. Ein xy-Scanner 9, der in einer Variante durch zwei im wesentlichen orthogonal ablenkende Galvanometerspiegel realisiert ist, lenkt den vom Laser 8 kommenden Laserstrahl zweidimensional ab, so daß nach dem xy-Scanner 9 ein abgelenkter Laserstrahl 10 vorliegt. Der xy-Scanner 9 bewirkt somit eine Verstellung der Lage des Fokus 7 im wesentlichen senkrecht zur Haupteinfallsrichtung der Laserstrahlung 2 in die Hornhaut 5. Zur Verstellung der Tiefenlage ist neben dem xy-Scanner 9 ein z-Scanner 11 vorgesehen, der beispielsweise als verstellbares Teleskop ausgebildet ist. Der z-Scanner 11 sorgt dafür, daß die z-Position der Lage des Fokus 7, d.h. dessen Position auf der optischen Achse des Einfalls verändert wird. Der z-Scanner 11 kann dem xy-Scanner 9 nach- oder vorgeordnet sein. Die nachfolgend mit x, y, z bezeichneten Koordinaten beziehen sich also auf die Ablenkung der Lage des Fokus 7.

Für die Definition der Punkte in der Hornhaut 5 ist die Zuordnung der einzelnen Koordinaten zu den Raumrichtungen nicht ausschlaggebend, der einfacheren Beschreibung halber ist jedoch nachfolgend mit z immer die Koordinate entlang der optischen Achse des Einfalls der Laserstrahlung 2 bezeichnet, und x sowie y bezeichnen zwei zueinander orthogonale Koordinaten in einer Ebene senkrecht zur Einfallsrichtung des Laserstrahls. Dem Fachmann ist natürlich bekannt, daß eine dreidimensionale Beschreibung der Lage der Punkte in der Hornhaut 5 auch durch andere Koordinatensysteme erfolgen kann, insbesondere muß es sich nicht um ein rechtwinkliges Koordinatensystem handeln. Daß der xy-Scanner 9 um zueinander rechtwinklige Achsen ablenkt ist ebenfalls nicht zwingend, vielmehr kann jeder Scanner verwendet werden, der in der Lage ist, den Fokus 7 in einer Ebene zu verstellen, in der die Einfallsachse der optischen Strahlung nicht liegt. Somit sind auch schiefwinklige Koordinatensysteme möglich.

Weiter können auch nicht-kartesische Koordinatensysteme zur Beschreibung bzw. Steuerung der Lage des Fokus 7 verwendet werden, wie dies nachfolgend auch noch erläutert wird. Beispiele für solche Koordinatensysteme sind Kugelkoordinaten (auch als sphärische Koordinaten bezeichnet) sowie zylindrische Koordinaten.

Zur Steuerung der Lage des Fokus 7 auf die Zielpunkte werden der xy-Scanner 9 sowie der z-Scanner 11, die gemeinsam ein konkretes Beispiel einer dreidimensionalen Fokusverstelleinrichtung realisieren, von einem Steuergerät 12 über nicht näher bezeichnete Leitungen angesteuert. Gleiches gilt für den Laser 8. Das Steuergerät 3 sorgt für einen geeignet synchronen Betrieb des Lasers 8 sowie der dreidimensionalen Fokusverstelleinrichtung, exemplarisch realisiert durch den xy-Scanner 9 sowie den z-Scanner 11, so daß die Lage des Fokus 7 in der Hornhaut 5 so verstellt wird, daß letztendlich ein Material bestimmten Volumens isoliert wird, wobei die spätere Volumenentfernung eine gewünschte Fehlsichtigkeitskorrektur bewirkt.

Das Steuergerät 12 arbeitet nach vorgegebenen Steuerdaten, welche die Zielpunkte für die Fokusverstellung vorgeben. Die Steuerdaten sind in der Regel in einem Steuerdatensatz zusammengefaßt. Dieser gibt in einer Ausführungsform die Koordinaten der Zielpunkte als Muster vor, wobei die Reihenfolge der Zielpunkte im Steuerdatensatz die Aneinanderreihung der Fokuslagen und damit letztlich eine Bahnkurve (hier auch verkürzt als Bahn bezeichnet) festlegt. Der Steuerdatensatz enthält in einer Ausführungsform die Zielpunkte als konkrete Stellwerte für den Fokuslagenverstellmechanismus, z.B. für den xy-Scanner 9 und den z-Scanner 11. Zur Vorbereitung des augenchirurgischen Verfahrens, also bevor das eigentliche Operationsverfahren ausgeführt werden kann, werden die Zielpunkte und vorzugsweise auch deren Reihenfolge im Muster bestimmt. Es muß eine Vorplanung des operativen Eingriffes dahingehend erfolgen, daß die Steuerdaten für das Behandlungsgerät 1 ermittelt werden, deren Anwendung dann eine für den Patienten 4 optimale Fehlsichtigkeitskorrektur erreicht.

Die Steuerdaten bewirken letztlich eine Erzeugung von Schnittflächen indem die Zielpunkte auf geeignete Art und Weise in der Hornhaut vorgegeben werden. Im Stand der Technik ist beispielsweise in der WO 2005/011546 zur Erzeugung von Schnittflächen in der Augenhornhaut beschrieben, daß spezielle Spiralen eingesetzt werden können, die beispielsweise um eine im wesentlichen senkrecht zur optischen Achse (z-Achse) liegende Hauptachse in Art einer Schraubenlinie verlaufen. Auch ist die Verwendung eines Scanmusters bekannt, das die Zielpunkte zeilenweise anordnet (vgl. WO 2005/011545). Diese Möglichkeiten können selbstverständlich zur Erzeugung der oben definierten Schnittflächen verwendet und mit den nachfolgend erläuterten Transformationen eingesetzt werden.

Die Verstellung der Lage des Fokus in der Augenhornhaut erfolgt mittels der in Figur 3 schematisch dargestellten dreidimensionalen Ablenkeinrichtung, die zur Verstellung des Fokus in z-Richtung die Verschiebung von Linsen oder anderer optisch wirksamer Elemente einsetzt.

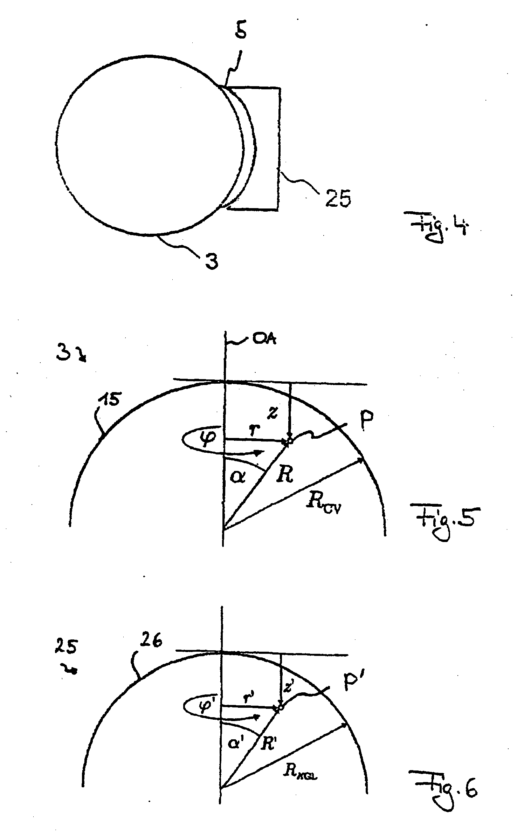

Bei der Bestimmung der Zielpunkte ist natürlich zu berücksichtigen, daß insbesondere bei einer Fehlsichtigkeitskorrektur letztendlich ein zu entnehmendes Volumen im Normalzustand des Auges definiert sein soll. Die letztlich interessierenden Schnittflächen betreffen deshalb das natürliche Auge. Es ist nun aber zu berücksichtigen, daß das Behandlungsgerät 1 aus Gründen der Fixierung des Auges mit einem Kontaktglas 25 arbeitet, das wie es in Figur 4 gezeigt ist, auf die Hornhautvorderfläche 15 der Augenhornhaut 5 aufgesetzt wird. Das Kontaktglas 25, das bereits Gegenstand mehrerer Patentpublikationen ist (exemplarisch sei beispielsweise auf die WO 2005/048895 verwiesen), ist für die hier vorliegende Beschreibung des Behandlungsgerätes 1 bzw. der damit in Zusammenhang stehenden Verfahren zur Vorbereitung und/oder Durchführung des chirurgischen Eingriffes allerdings nur insoweit von Interesse, als es der Hornhautvorderfläche 15 eine definierte Krümmung verleiht. Hinsichtlich der sphärischen Krümmung der Kontaktfläche des Kontaktglases 25 unterscheidet sich der hier beschriebene Ansatz jedoch deutlich von dem Ansatz, wie er beispielsweise in der WO 2003/002008 beschrieben ist, der ein planes Kontaktglas verwendet, welches die Augenhornhaut flachdrückt.

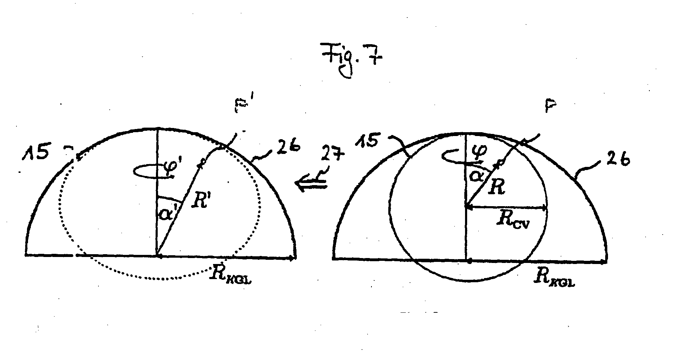

Wird das Auge an das Kontaktglas 25 mit sphärischer Kontaktfläche angepreßt, kommt es zu einer räumlichen Deformation des Auges. Das Anpressen entspricht einer Transformation vom Koordinatensystem des Auges, wie es exemplarisch in Figur 5 dargestellt ist, in das Koordinatensystem des Kontaktglases, das beispielshalber in Figur 6 gezeigt ist. Dieser Zusammenhang ist dem Fachmann aus der WO 2005/011547 bekannt, deren Offenbarungsgehalt diesbezüglich vollumfänglich eingebunden sein soll. In den Figuren 5 und 6 bezeichnen mit einem Apostroph versehene Koordinaten die Koordinaten des auf das Kontaktglas 25 bzw. dessen dem Auge zugewandte Kontaktglasunterseite 26 bezogene Größen. Ein beliebiger Punkt P in der freien Hornhaut (Fig. 5) entspricht dann einem Punkt P' in der an die Kontaktfläche 25 angedrückten Hornhaut (Fig. 7, links).

Das Kontaktglas hat aber noch einen weiteren Vorteil. Durch das Anpressen an die sphärische Kontaktglasunterseite 26 ist automatisch auch die Hornhautvorderfläche 15 sphärisch. Eine in konstantem Abstand unter der Hornhautvorderfläche 15 liegende Fläche ist damit bei angepreßtem Kontaktglas ebenfalls sphärisch, was zu erheblich vereinfachter Ansteuerung führt. Es ist deshalb bislang immer versucht worden, ein Kontaktglas 25 mit sphärischer Kontaktglasunterseite 26 zu verwenden und zumindest für eine Schnittfläche Zielpunkte vorzugeben, die diese Schnittfläche als sphärische Fläche in konstantem Abstand unter der Hornhautvorderfläche 15 definieren.

Die Darstellungen in den Figuren 5 und 6 zeigen die Koordinatentransformation, die am Auge durch das Aufsetzten bzw. Abnehmen des Kontaktglases auftritt. Sie enthalten sowohl Kugelkoordinaten $(R, \alpha, \varphi)$ bezogen auf den Ursprung der gekrümmten Fläche (Hornhautvorderfläche 15 bzw. Kontaktglasunterseite 26) als auch Zylinderkoordinaten $(R, z, \varphi)$ bezogen auf den durch den Durchtrittspunkt der optischen Achse OA definierten Scheitelpunkt der Hornhautvorderfläche 15 bzw. der Kontaktglasunterseite 26.

Eine Koordinatentransformation tritt aber ganz unabhängig von den gewählten Koordinatensystemen auf, wenn ein Punkt in der Hornhaut im freien (oder angepreßten) Auge gegeben ist und im angepreßten (oder freien) Auge beschrieben werden soll.

Bei der Koordinatentransformation vom auf das freie Auge bezogenen Koordinatensystem, wie es in Figur 7 dargestellt ist, in das auf das Kontaktglas bezogene System des angepreßten Auges gemäß Figur 7 bleiben die Bogenlänge, d.h. $\alpha \cdot R$, die radiale Tiefe $(R_{cv} - R)$, sowie der Winkel $\varphi$ erhalten. Die Transformation von für das natürliche Auge, d.h. im Koordinatensystem der Figur 7, zugrundegelegten Zielpunkten ist somit ein wichtiger Schritt bei der Berechnung der Ansteuergrößen für die dreidimensionale Fokusverstelleinrichtung. Sie verläuft grundsätzlich anders als bei einem ebenen Kontaktglas, in dem z.B. eine sphärische Fläche zu einer Ebene entartet.

Das Anpressen der Hornhaut 5 des Auges 3 an die sphärisch gekrümmte Kontaktglasunterseite 26 ist in Figur 7 veranschaulicht. Dort zeigt die rechte Darstellung schematisch den Zustand, wenn die Kontaktglasunterseite 26 nur am Scheitelpunkt in Kontakt mit der Hornhautvorderfläche 15 ist. Die Hornhaut ist noch unverformt. Zur Verdeutlichung der geometrischen Beziehungen ist die Hornhautvorderfläche 15 schematisch in Figur 7 als Kreis eingezeichnet. Das Anpressen des Kontaktglases 25 auf die Hornhaut 5 bewirkt den durch Pfeil 27 symbolisierten Übergang zum Zustand der linken Seite der Figur 7. Das Abnehmen des Kontaktglases 25 bewirkt eine Relaxation des Auges 3 entgegen der Richtung des Pfeiles 27 Aufgrund der geschilderten Rahmenbedingungen transformieren sich für jeden Punkt in der Augenhornhaut 5 die Koordinaten von dem in Figur 5 dargestellten System in das System der Figur 6. Da das Anlegen der Hornhautvorderfläche 15 in der Regel durch Ansaugen mittels Unterdruck bewirkt wird, wird die Transformation nachfolgend auch als Ansaugtransformation bezeichnet.

Folgendes Vorgehen ermöglicht eine allgemeine Transformation der Koordinaten eines Punktes P des entspannten Auges (nichtangesaugt) in Koordinaten für einen entsprechenden Punkt P' des angesaugten Auges. Die Rücktransformation wird anschließend beschrieben. Dieser allgemeine Ansatz benötigt keine spezielle Geometrie des Kontaktglases oder der Kornea-Vorderfläche, verwendet aber eine Kornea-Vorderfläche, welche die anteriore Oberfläche der Kornea ohne einen Tränenfilm ist:

1. Numerische oder analytische Beschreibung der Vorderfläche 15 der Kornea in einem beliebigen Koordinatensystem, bei experimenteller Bestimmung ggf. unter Anwendung geeigneter Glättungsverfahren. Bei Berücksichtigung einer Lage der Biegelinie (bzw. neutralen Phase) im Abstand L unter der Vorderfläche 15 der Cornea wird bei der Ansaugtransformation statt der Vorderfläche 15 eine Bezugsfläche V verwendet, welche gegenüber der Vorderfläche 15 um L radial kontrahiert ist. Wenn die Biegelinie nicht berücksichtigt werden soll, ist die Bezugsfläche V gleich der Vorderfläche 15 der Hornhaut 5.

2. Numerische oder analytische Beschreibung der Kontaktfläche 26 des Kontaktglases 25 in einem beliebigen Koordinatensystem, bei experimenteller Bestimmung ggf. unter Anwendung geeigneter Glättungsverfahren. Die Kontaktfläche 26 wird in der Transformation als diejenige Fläche betrachtet, die die Vorderfläche 15 der Hornhaut

im angepreßten Auge annimmt. Bei Berücksichtigung einer Flüssigkeitsfilmdicke F' und/oder Biegelinienverschiebung L ist jedoch anstelle der Kontaktfläche 26 eine Kontaktglas-Bezugsfläche G zu verwenden, die gegenüber der Kontaktfläche 26 um F' + L radial kontrahiert ist. Falls Flüssigkeitsfilmdicke F' und Biegelinienverschiebung L nicht berücksichtigt werden sollen, fallen auch hier Kontaktfläche 26 und Kontaktglas-Bezugsfläche G zusammen.

3. Bestimmung des in der Bezugsfläche V liegenden Ursprungspunktes A der Transformation, der bei der Transformation seine Koordinaten nicht ändert. Dieser Punkt ist vorzugsweise der Schnittpunkt von Sehachse und Bezugsfläche V oder der geometrische Scheitelpunkt der Bezugsfläche V. Beide Punkte werden hier unter dem Begriff "Scheitelpunkt" zusammengepaßt.

4. Bestimmung des Punktes M auf der Kontaktglas-Bezugsfläche G, der nach dem Ansaugen die geringste Entfernung zum Ursprungspunkt A hat. Es gilt in guter Näherung A =M.

5. Bestimmung aller Punkte $O_i$ ($\varphi$) auf der Bezugsfläche V, auf deren Flächennormalen der Punkt P ($\varphi$) liegt. Die Flächennormale sei hier als Gerade verstanden, welche senkrecht auf der Bezugsfläche V steht und die Punkte $O_i$ beinhaltet.

6. Bestimmung des Punktes $O \in O_i$ für den die Strecke $\overline{PO_i}$ ein Minimum ist. Dies ist also der Schnittpunkt zwischen der Flächennormalen und der Bezugsfläche V.

7. Berechnung der Kurvenlänge $B_\varphi = \int_{O(\varphi)}^{A} V$ von O zu A in der durch den Zylinderwinkel $\varphi$ charakterisierten Schnittebene.

8. Berechnung des Punktes $O' \in G$ für den gilt: $B_\varphi = \int_{O'(\varphi)}^{M} G$.

9. Berechnung des Punktes P'($\varphi$) für den gilt: $\overline{P'O'}$ und $\overline{PO}$

10. Es gilt stets $\varphi' = \varphi$.

Die Rücktransformation geschieht mit folgenden Schritten:

1. Numerische oder analytische Beschreibungen analog zu obigen Punkten 1 und 2.

2. Für den Ursprungspunkt wird das unter obiger Nr. 3 erwähnte Vorgehen verwendet.

3. Bestimmung des Punktes M auf der Kontaktglas-Bezugsfläche G, der die geringste Entfernung zum Ursprungspunkt A der Transformation hat. Es gilt in guter Näherung A = M.

4. Bestimmung aller Punkte $O_i'$ ($\varphi$) auf der Kontaktglas-Bezugsfläche G auf deren Flächennormalen der Punkt P' ($\varphi$) liegt. Die Flächennormale ist wiederum die Gerade, welche senkrecht auf der Kontaktglas-Bezugsfläche G steht und Punkt $O_i'$ beinhaltet.

5. Bestimmung des Punktes $O' \in O_i'$ für den die Strecke $\overline{P'O_i'}$ ein Minimum ist.

6. Berechnung der Kurvenlänge $B'_\varphi = \int_{O'(\varphi)}^{A'} G$ von O' zu A' in der durch den Zylinderwinkel $\varphi$ charakterisierten Schnittebene.

7. Berechnung des Punktes $O \in V$ für den gilt: $B'_\varphi = \int_{o'(\varphi)}^{M} V$.

8. Berechnung des Punktes P($\varphi$) für den gilt: $\overline{P'O'} = \overline{PO}$

9. Es gilt stets: $\varphi' = \varphi$.

Mit dem obigen Verfahren läßt sich für beliebige Flächenformen sowohl der Hornhautvorderfläche 15 als der Kontaktglas-Kontaktfläche 26 die Ansaugtransformation durchführen.

Wenn die zu transformierenden Punkte in einem Abstand zur jeweiligen Bezugsfläche liegen, der kleiner ist als der lokale Krümmungsradius der Bezugsfläche, können die Punkte Nr. 5 der Hin-Transformation und Nr. 4 der Rück-Transformation entfallen. Der lokale Krümmungsradius ergibt sich aus dem Radius einer bestangepaßten Sphäre am entsprechenden Punkt und kann vereinfacht mit dem kleinsten Krümmungsradius der Fläche angenähert werden. Bei der Augenchirurgie ist die Bedingungen zum Entfall der Punkte Nr. 5 bzw. Nr. 4 meist gegeben, da die Hornhaut deutlich dünner, als ihr Krümmungsradius ist.

Für die spezielle Bedingung einer paraboloidischen Oberfläche der Kornea-Vorderseite und sphärische Kontaktglasform, welche also im radialen Schnittbild Parabeln zeigt, gibt es eine spezielle Lösung. Eine Parabel entspricht der natürlichen Form der Kornea 5 noch etwas besser als die im Stand der Technik bereits bekannte Lösung für eine beliebige Sphäre.

Eine besonders rechensparende Vereinfachung dieser speziellen Lösung liefert folgende Erweiterung der aus WO 2005/011547 bekannten Ansaugtransformationen für eine sphärische Kornea-Vorderfläche an sphärischem Kontaktglas auf eine Paraboloid-förmige Hornhautvorderfläche:

$$\varphi = \varphi'$$

$$\alpha' \cdot (R' - F' - L) = (R - L) \cdot (\alpha + \frac{c_5}{40} \alpha^5 - \frac{c_7}{84} \alpha^7 + K_1)$$

$$R' = R_{KGL} + R - R_{CV} (1 + \frac{f_4}{8} \alpha^4 - \frac{f_6}{12} \alpha^6 + K_2)$$

Der bekannte sphärische Ansatz wird also modifiziert, um Abweichungen von der ideal sphärischen Form der Vorderfläche 15 der Kornea 5 (bzw. des Kontaktglases 25) hin zu einer Parabel zu berücksichtigen.

Mitunter bedürfen die analytischen hergeleiteten Vorfaktoren einer weiteren Anpassung aufgrund experimenteller Daten. Die Vorfaktoren $c_i$, $f_i$ können also experimentelle bestimmte Werte ungleich 1 zugewiesen bekommen, um Abweichungen der Hornhaut-Vorderfläche 15 von einem Paraboloid in Richtung auf eine Sphäre oder ein Ellipsoid zu berücksichtigen.

Wenn die Flüssigkeitsfilmdicke F' im angepreßten Zustand und eine von der Vorderfläche 15 der Kornea 5 um einen Abstand L nach innen verschobene Biegelinie berücksichtigt werden soll, so kann die Ansaugtransformation für ein sphärisches Kontaktglas und eine sphärische Hornhautvorderfläche in der folgenden Form geschrieben werden.

$$\varphi = \varphi'$$

$$\alpha' \cdot (R_{KGL} - F' - L) = \alpha \cdot (R_{CV} - L)$$

$$R_{KGL} - R' = R_{CV} - R$$

Viele Meßgeräte messen den Krümmungsradius der Kornea-Vorderfläche 15 einschließlich einer Dicke T des natürlichen Tränenfilms, liefern also direkt den Wert $R_{CV} + T$, welcher entsprechend korrigiert werden muß, da obige Gleichungen von $R_{CV}$ ausgehen. Es sei angemerkt, daß dieser Ansatz selbstverständlich mit den beschriebenen Transformationen kombiniert werden kann. Auch kann T, F und/oder L zur Vereinfachung vernachlässigt werden.

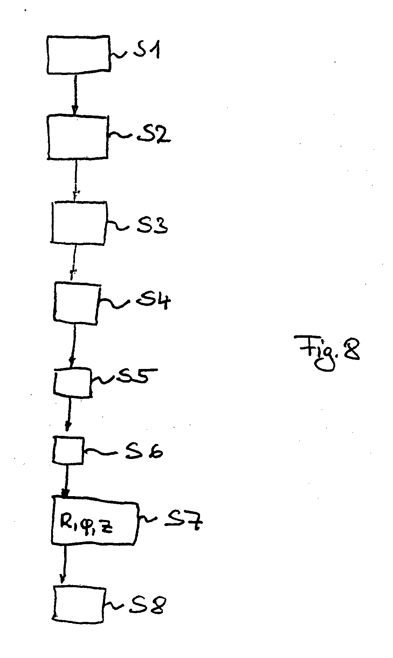

Der Ablauf der Vorbereitung des Gerätes 1 auf den Einsatz bei einer augenchirurgischen Fehlsichtigkeitsoperation ist schematisch in Figur 8 zusammengefaßt. In einem Schritt S1 erfolgt die Vermessung des Auges 3. Dabei werden für die beim Patienten 4 vorliegenden Fehlsichtigkeit Korrekturparameter erhalten. Die in Schritt S2 aufgestellten Parameter werden dann in einem Schritt S3 dazu verwendet, die zur Korrektur nötige neue Krümmung der Hornhaut 5 zu bestimmen. Ist diese Berechnung in Schritt S3 abgeschlossen, wird das Volumen in S4 bestimmt, das für die Krümmungsänderung aus der Hornhaut entnommen werden muß. Dazu werden Schnittflächen in einem Schritt S5 festgelegt, die das Volumen umgrenzen. Hat man die entsprechenden Funktionsbeschreibungen dieser Flächen gewonnen, wird in Schritt S6 die Ansaugtransformation, die sich beim Ansaugen des Auges an das Kontaktglas auswirkt, berücksichtigt. Hierbei wird einer der oben geschilderten Zusammenhänge eingesetzt.

Als nächstes werden die Koordinaten der Bahnkurven ermittelt, aus denen die Schnittflächen aufgebaut werden. Dies ist schematisch in Schritt S7 durch die Parameter R, $\varphi$, z angedeutet. Am Ende des Schrittes S7 liegt ein Punkt-Muster mit den Koordinaten der Spots, auf die jeweils ein Laserstrahlungspuls einwirken soll. Die Dichte der Zielpunkte kann dabei bereits zur Vereinfachung des rechnerischen Aufwandes reduziert sein.

Mit den derart ermittelten Ansteuerparametern wird dann in Schritt S8 die eigentliche Operation durchgeführt und das zu entfernende Volumen durch die Schnittflächen umgrenzt.

## Patentansprüche

1. Verfahren zum Erzeugen von Steuerdaten für eine augenchirurgische Behandlungsvorrichtung (1), welche mittels einer Lasereinrichtung (E) in der Augen-Hornhaut (5) Gewebeschichten trennt, wobei im Betrieb der Lasereinrichtung (E) ein eine Kontaktfläche (26) aufweisendes Kontaktglas (25) die Hornhaut (5) in die Form der Kontaktfläche verformt, wozu die Kontaktfläche gegen die Hornhaut gedrückt wird, wobei das Verfahren folgende Schritte umfaßt:

   - die Steuerdaten für die Lasereinrichtung (E) werden so erzeugt, daß sie Koordinaten von in der Hornhaut (5) liegenden Zielpunkten (28) für die Lasereinrichtung (E) vorgeben, und
   - bei der Erzeugung der Zielpunkt-Koordinaten wird die Verformung der Hornhaut (5) berücksichtigt, welche während des Betriebs der Lasereinrichtung (E) bedingt durch das Kontaktglas (25) vorliegt,
   **dadurch gekennzeichnet, daß**
   bei der Berücksichtigung der Verformung folgende Schritte ausgeführt werden, um eine verformungsbedingte Verschiebung eines Punktes P in der unverformten Hornhaut (5) zu ermitteln:

   1a) zum Punkt P wird ein Punkt O auf einer Bezugsfläche V der unverformten Hornhaut (5) ermittelt, der so liegt, daß die durch den Punkt O laufende Flächennormale durch den Punkt P geht, wobei die Bezugsfläche V entweder die Vorderfläche (15) selbst oder eine durch radiale Kontraktion der Vorderfläche (15) um eine Biegelinienverschiebung L erhaltene Fläche ist,
   1b) ein Abstand $\overline{SO}$ zwischen einem Scheitelpunkt S der Bezugsfläche (V) und dem Punkt O wird ermittelt, wobei der Abstand $\overline{SO}$ die Bogenlänge auf der Bezugsfläche (V) ist,
   1c) es wird ein Punkt O' auf einer Kontaktglas-Bezugsfläche (G) ermittelt, der im Abstand $\overline{SO}$ vom Kontaktflächenscheitel liegt, wobei die Kontaktglas-Bezugsfläche (G) entweder die Kontaktfläche (26) selbst oder eine durch radiale Kontraktion der Kontaktfläche (26) um die Biegelinienverschiebung L und/oder eine Dicke F' eines Flüssigkeitsfilms auf der Hornhaut (5) erhaltene Fläche ist,
   1d) auf einer Flächennormalen im Punkt O' wird ein Punkt P' ermittelt, der denselben Abstand von O' hat, wie der Punkt P vom Punkt O, und
   1e) der Punkt P' wird als durch die Verformung verschobener Punkt P verwendet;

   und/oder bei der Berücksichtigung der Verformung folgende Schritte ausgeführt werden, um eine relaxationsbedingte Verschiebung eines Punktes Q' in der verformten Hornhaut zu ermitteln:

   2a) zum Punkt Q' wird ein Punkt O' auf der Kontaktglas-Bezugsfläche (G) ermittelt,
   der so liegt, daß die durch den Punkt O' laufende Flächennormale durch den Punkt Q' geht,
   2b) ein Abstand $\overline{SO'}$ zwischen dem Scheitelpunkt S und dem Punkt O' wird ermittelt, wobei der Abstand $\overline{SO'}$ die Bogenlänge auf der Kontaktglas-Bezugsfläche (G) ist,
   2c) es wird ein Punkt O auf der Bezugsfläche (V) ermittelt, der im Abstand $\overline{SO'}$ vom Scheitelpunkt S liegt,
   2d) auf einer Flächennormalen im Punkt O wird ein Punkt Q ermittelt, der denselben Abstand von O hat, wie der Punkt Q' vom Punkt O', und
   2e) der Punkt Q wird als durch die Relaxation verschobener Punkt Q' verwendet.

2. Verfahren nach Anspruch 1, **dadurch gekennzeichnet, daß** die Kontaktfläche (26) nicht-sphärisch ist.

3. Verfahren nach Anspruch 2, **dadurch gekennzeichnet, daß** die Kontaktfläche (26) nichtrotationssymmetrisch ist.

4. Augenchirurgische Behandlungsvorrichtung, welche aufweist:

- eine Lasereinrichtung (E) zur Trennung von Gewebeschichten der Augen-Hornhaut (5),
- ein eine Kontaktfläche aufweisendes Kontaktglas (25), das im Betrieb der Lasereinrichtung (E) die Hornhaut (5) in die Form der Kontaktfläche verformt, wozu die Kontaktfläche zuerst mit einem Kontaktflächenscheitel auf einen Hornhautscheitel gesetzt und dann zur Verformung der Hornhaut gegen diese gedrückt wird,
- ein Steuergerät, das Steuerdaten für die Lasereinrichtung (E) so erzeugt, daß sie Koordinaten von in der Hornhaut (5) liegenden Zielpunkt (28) für die Lasereinrichtung (E) vorgeben, und bei der Erzeugung der Zielpunkt-Koordinaten die Verformung der Hornhaut (5) berücksichtigt, welche während des Betriebs der Lasereinrichtung (E) bedingt durch das Kontaktglas (25) vorliegt,
**dadurch gekennzeichnet, daß**
das Steuergerät dazu ausgebildet ist, das Verfahren gemäß einem der obigen Verfahrensansprüche auszuführen.

## Claims

1. Procedure for generating control data for an eye-surgery treatment apparatus (1) that, by means of a laser device (E), separates tissue layers in the cornea (5) of the eye, wherein, during operation of the laser device (E), a contact glass (25) having a contact surface (26) deforms the cornea (5) into the shape of the contact surface, for which purpose the contact surface is pressed against the cornea, the procedure comprising the following steps:

the control data for the laser device (E) are generated in such a way that they specify coordinates of target points (28) for the laser device (E) that are located in the cornea (5) and, - in the generation of the target-point coordinates, the deformation of the cornea (5), caused by the contact glass (25), that exists during the operation of the laser device (E) is considered,
**characterised in that**
the following steps are executed in the consideration of the deformation, in order to determine a displacement of a point P in the non-deformed cornea (5), the displacement being caused by deformation:

1a) on a reference surface V of the non-deformed cornea (5) there is determined, in relation to the point P, a point O that is located in such a way that the surface normal running through the point O goes through the point P, the reference surface V being either the anterior surface (15) itself or a surface obtained through radial contraction of the anterior surface (15) by a bend line displacement L,
1b) a distance $\overline{SO}$ between a vertex S of the reference surface (V) and the point O is determined, the distance $\overline{SO}$ being the arc length on the reference surface (V), 1c) on a contact-glass reference surface (G) there is determined a point O' that is located at the distance $\overline{SO}$ from the contact-surface vertex, the contact-glass reference surface (G) being either the contact surface (26) itself or a surface obtained through radial contraction of the contact surface (26) by the bend line displacement L and/or by a thickness F' of a fluid film on the cornea (5),
1d) on a surface normal in the point O' there is determined a point P' that has the same distance from O' as the point P has from the point O, and
1e) the point P' is used as the point P displaced by the deformation;

and/or the following steps are executed in the consideration of the deformation, in order to determine a displacement of a point Q' in the deformed cornea, the displacement being caused by relaxation:

2a) on the contact-glass reference surface (G) there is determined, in relation to the point Q', a point O' that is located in such a way that the surface normal running through the point O' goes through the point Q',
2b) a distance $\overline{SO'}$ between the vertex S and the point O' is determined, the distance $\overline{SO'}$ being the arc length on the contact-glass reference surface (G),
2c) on the reference surface (V) there is determined a point O that is located at the distance $\overline{SO'}$ from the vertex S,
2d) on a surface normal in the point O there is determined a point Q that has the same distance from O as the point Q' has from the point O', and
2e) the point Q is used as the point Q' displaced by the relaxation.

**2.** Procedure according to claim 1, **characterised in that** the contact surface (26) is non-spherical.

**3.** Procedure according to claim 2, **characterised in that** the contact surface (26) is non-rotationally symmetrical.

**4.** Eye-surgery treatment apparatus, which comprises:

- a laser device (E) for separating tissue layers of the cornea (5) of the eye,
- a contact glass (25), which has a contact surface and which, during operation of the laser device (E), deforms the cornea (5) into the shape of the contact surface, for which purpose the contact surface is first set, with a contact-surface vertex, onto a corneal vertex and, for the purpose of deforming the cornea, is then pressed against the latter,
- a control device, which generates control data for the laser device (E) in such a way that the control data specify coordinates of target points (28) for the laser device (E) that are located in the cornea (5), and which, in the generation of the target-point coordinates, considers the deformation of the cornea (5), caused by the contact glass (25), that exists during the operation of the laser device (E),
**characterised in that**
the control device is configured to execute the procedure according to any one of the above procedure claims.

**Revendications**

**1.** Procédé de production de données de commande pour un dispositif de traitement pour la chirurgie de l'oeil (1) qui découpe des couches de tissu dans la cornée (5) au moyen d'un dispositif laser (E), étant entendu que dans le cadre du fonctionnement du dispositif laser (E), un verre de contact (25) présentant une surface de contact (26) déforme la cornée (5) dans la forme de la surface de contact en ce que la surface de contact est pressée contre la cornée, étant entendu que le procédé comprend les étapes suivantes :

- les données de commande pour le dispositif laser (E) sont produites de telle sorte qu'elles définissent des coordonnées de points visés (28) situés dans la cornée (5) pour le dispositif laser (E), et
- la déformation de la cornée (5) qui intervient lors de l'opération du dispositif laser (E) sous l'effet du verre de contact (25) est prise en considération dans la production des coordonnées des points visés, **caractérisé en ce que** les étapes suivantes sont exécutées aux fins de la prise en considération de la déformation afin de déterminer un décalage dû à la déformation d'un point P dans la cornée (5) non déformée :

1a) par rapport au point P, on détermine un point O sur une surface de référence V de la cornée non déformée (5) qui est situé à un endroit tel que la normale à la surface traversant le point O passe à travers le point P, étant entendu que la surface de référence V est soit la surface avant (15) elle-même, soit une surface obtenue par une contraction radiale de la surface avant (15) correspondant à un décalage de la ligne de flexion L,
1b) on détermine une distance $\overline{SO}$ entre un point de sommet S de la surface de référence (V) et le point O, étant entendu que la distance $\overline{SO}$ est la longueur de l'arc sur la surface de référence (V),
1c) on détermine un point O' sur une surface de référence du verre de contact (G), lequel est situé à la distance $\overline{SO}$ du sommet de la surface de contact, étant entendu que la surface de référence du verre de contact (G) est soit la surface de contact (26) elle-même, soit une surface obtenue par une contraction radiale de la surface de contact (26) correspondant à un décalage de la ligne de flexion L et/ou à une épaisseur F' d'une pellicule liquide sur la cornée (5),
1d) on détermine, sur une normale à la surface du point O', un point P' qui est situé à la même distance de O' que le point P par rapport au point O, et
1e) le point P' est utilisé comme point P décalé sous l'effet de la déformation ;

et/ou en ce que les étapes suivantes sont exécutées aux fins de la prise en considération de la déformation afin de déterminer un décalage dû au relâchement d'un point Q' dans la cornée déformée :

2a) par rapport au point Q', on détermine un point O' sur la surface de référence du verre de contact (G) qui est situé à u endroit tel que la normale à la surface traversant le point O' passe à travers le point Q',
2b) on détermine une distance $\overline{SO'}$ entre le point de sommet S et le point O', étant entendu que la distance $\overline{SO'}$ est la longueur de l'arc sur la surface de référence du verre de contact (G),
2c) on détermine un point O sur la surface de référence (V), lequel est situé à la distance $SO'$ du point de sommet S,

2d) on détermine, sur une normale à la surface du point O, un point Q qui est situé à la même distance de O que le point Q' par rapport au point O', et

2e) le point Q est utilisé comme point Q' décalé sous l'effet du relâchement.

**2.** Procédé selon la revendication 1, **caractérisé en ce que** la surface de contact (26) n'est pas sphérique.

**3.** Procédé selon la revendication 2, **caractérisé en ce que** la surface de contact (26) n'est pas symétrique en rotation.

**4.** Dispositif de traitement pour la chirurgie de l'oeil, qui comprend :

- un dispositif laser (E) destiné à la découpe de couches de tissu de la cornée (5),
- un verre de contact (25) présentant une surface de contact qui, dans le cadre du fonctionnement du dispositif laser (E), déforme la cornée (5) dans la forme de la surface de contact en ce que la surface de contact est d'abord placée sur un sommet de la cornée avec un sommet de la surface de contact, puis pressée contre la cornée de façon à la déformer,
- un appareil de commande qui produit des données de commande pour le dispositif laser (E) de telle sorte qu'elles définissent des coordonnées d'un point visé (28) situé dans la cornée (5) pour le dispositif laser (E) et qui, lors de la production des coordonnées des points visés, prend en considération la déformation de la cornée (5) qui intervient lors de l'opération du dispositif laser (E) sous l'effet du verre de contact (25),

**caractérisé**

**en ce que** l'appareil de commande est conçu pour exécuter le procédé selon l'une des revendications précédentes.

FIG 1

FIG 2

Fig. 1a

FIG 3

5

25

3

Fig. 4

3↘

15

OA

φ

r

z

α

R

P

R_{CV}

Fig. 5

25

26

φ'

r'

z'

α'

R'

P'

R_{KGL}

Fig. 6

Fig. 7

Fig. 8

**EP 2 285 322 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5984916 A
- WO 2005011545 A1
- WO 2005011547 A1
- WO 200501157 A1
- EP 1159986 A1
- US 5549632 A
- DE 69500997 T2
- WO 2005011546 A
- WO 2005011545 A
- WO 2005048895 A
- WO 2003002008 A
- WO 2005011547 A